(19) **Europäisches Patentamt / European Patent Office / Office européen des brevets**

(11) **EP 1 485 696 B1**

(12) **EUROPEAN PATENT SPECIFICATION**

(45) Date of publication and mention
of the grant of the patent:
**26.03.2008 Bulletin 2008/13**

(51) Int Cl.:
***G01N 21/00*** *(2006.01)*

(21) Application number: **03725985.0**

(22) Date of filing: **07.02.2003**

(86) International application number:
**PCT/US2003/003852**

(87) International publication number:
**WO 2003/073075 (04.09.2003 Gazette 2003/36)**

(54) **METHOD FOR THE ANALYSIS OF LUBRICANT BASICITY**

VERFAHREN ZUR ANALYSE DER BASIZITÄT VON SCHMIERMITTELN

PROCEDE D'ANALYSE DE LA BASICITE D'UN LUBRIFIANT

(84) Designated Contracting States:
**DE ES FR GB IT NL**

(30) Priority: **26.02.2002 US 361375 P**
**24.01.2003 US 350556**

(43) Date of publication of application:
**15.12.2004 Bulletin 2004/51**

(73) Proprietor: **ExxonMobil Research and**
**Engineering Company**
**Annandale, NJ 08801-0900 (US)**

(72) Inventors:
• **REISCHMAN, Paul, Thomas**
**Lambertville, NJ 08530 (US)**
• **CAREY, Vincent, Mark**
**Sewell, NJ 08080 (US)**
• **KELLY, Kevin, John**
**Mullica, NJ 08062 (US)**

(74) Representative: **Troch, Geneviève et al**
**ExxonMobil Chemical Europe Inc.,**
**P.O. Box 105**
**1830 Machelen (BE)**

(56) References cited:
| | |
|---|---|
| **EP-A- 0 695 798** | **GB-A- 2 180 339** |
| **US-A- 4 780 224** | **US-A- 5 250 204** |
| **US-A- 5 470 495** | **US-A- 5 569 842** |

## Description

### FIELD OF THE INVENTION

The present invention relates to a process for the measurement of a lubricant's basicity. More specifically, the present invention relates to a method to directly measure a lubricating oil's Total Base Number (TBN) during engine operations using an infrared ("IR") sensing device.

### BACKGROUND OF THE INVENTION

In internal combustion engines, lubricant oils have been used to lubricate piston rings, cylinder liners, bearings for crank shafts and connecting rods, valve train mechanisms including cams and valve lifters, among other moving members. In addition to the lubricating purposes listed above, lubricating oils are also used for cooling engines, neutralizing and dispersing combustion products, preventing rust and corrosion, and preventing deposits.

Lubricant oils for internal combustion engines are required to exhibit a variety of functions. As engines produce higher power and are operated under more severe conditions, the lubricating oil's required functionality has dramatically increased. These increased performance demands have resulted in a corresponding increase in the lubricant's expense. Lubricants are being made with increasingly sophisticated and expensive base stocks, including wholly synthetic base stocks. In addition, a wide variety of expensive additives, such as dispersants, detergents, antiwear agents, friction reducing agents and antioxidants are incorporated into the lubricants to meet functional demands.

One particularly important function of lubricating oils is to prevent the corrosion and wear of the lubricated components. These processes are caused or enhanced by acids found in an engine's fuel or produced during the combustion of that fuel. In particular, sulfur in fuel is converted to sulfur oxides during combustion. A fraction of these oxides upon contact with liquid water form damaging sulfuric acid. Similarly, nitrogen from air can be oxidized during combustion to form nitrogen oxides. A fraction of these oxides upon contact with liquid water form nitric acid. Both acids significantly increase the wear and corrosion of the engine components.

Lubricant oils are formulated to be highly alkaline in order to neutralize the acids formed during the combustion process. Indeed, lubricant additives are routinely "overbased" - a process that causes the additive to be able to neutralize far more acid than would be possible with its core chemical constituent. Such overbasing processes are described in the Kirk-Othmer Encyclopedia of Chemical Technology, 3d ed., Vol. 8, p.39.

A measure of a lubricant's alkalinity is the Total Base Number ("TBN"). Of late, this has also been referred to as simply the Base Number ("BN"), which shall be considered interchangeable with TBN herein. Certain levels of TBN are required for many types of engine oils. For example, modern locomotive engines require a TBN level of at least 13. OEMs of marine diesel engines either require or strongly recommend lubricants with TBN levels ranging from 5 to 70 depending on the range of sulfur levels in the fuel being burned and on the function of the lubricant. TBN is reported in terms of milligrams of potassium hydroxide per gram (mg KOH/g) of lubricant, reflecting the acid neutralizing capacity relative to the strong base potassium hydroxide. Standard laboratory methods for determining TBN are ASTM D2896 and D4739. Both are potentiometric titrations with the former using perchloric acid and the latter using hydrochloric acid. The conventional method used in the marine industry for both new and used oils is D2896. Such method is used in US 5250204 and EP 695798.

Alternate quicker methods may be used. For example, one method adds dilute acid to the lubricant in a closed reaction vessel. The acid reacts with the base in the lubricant. The reacted overbase carbonate forms carbonic acid which then degrades to carbon dioxide and water. The increase in pressure in the reaction vessel due to the carbon dioxide formation roughly correlates to the TBN of the lubricant when the pressure and TBN for a new oil sample are known (see for example GB 2180338).

Another method adds trifluoroacetic acid to the lubricant oil. An IR device measures the absorbance band due to the trifluoroacetate salt resulting from acid neutralization. Trifluoroacetate is easily distinguishable from other carboxylate groups because the highly electronegative fluorine shifts the peak to higher frequencies relative to carboxylates with no fluorine such as those found in oxidized lubricants.

All of these methods employ an acid reagent to measure TBN. For on-line or near real-time measurements, the complexity of adding reagents is undesirable. Further, adding an acid to the lubricant in or near an operating engine degrades lubricant functionality, is a more complex procedure and could lead to spills with undesirable consequences. Thus, neither of these methods would be useful for real time or near real time monitoring of a lubricant's TBN in an operating system.

Infrared analysis are used to determine the progress of chemical reactions. In US 5470495 it is used to confirm the consumption of carbonate during the formation of colloidal products, in US 478224 it is used to indicate the presence of amorphous and crystalline calcium carbonate and in US 5569842 it is used for analyzing one or more properties indicative of the conditions of the lubricant such as for example the lubricant degradation, the lubricant contamination or the additive depletion.

None of these documents involve the TBN determination.

Yet, a need for the real time or near real time *in situ* determination of TBN has developed because it has been discovered that the *in situ* TBN of an engine's lubricant, when combined with certain other parameters, accurately predicts the instantaneous wear of the engine components. The present invention demonstrates that a lubricant's TBN can be directly measured *in situ* without the addition of such a reagent.

## SUMMARY OF THE INVENTION

The present invention relates to a method for the measurement of a lubricating oil's Total Base Number during engine operation.
Specifically, the present invention relates to a method of directly monitoring the TBN of a lubricating oil by measuring one or more pre-selected Infrared ("IR") absorption bands.

## BRIEF DESCRIPTION OF THE DRAWINGS

Figure 1 is a table detailing the results for 13 samples for which TBN was measured by ASTM method D2896 and the corresponding carbonate and sulfate absorbance spectra ranges and values.

Figure 2 graphically illustrates the correlation of Infrared peak absorbance at about 863 cm$^{-1}$ correlating to carbonate species as compared to the ASTM D2896 TBN of various lubricant oils.

Figure 3 graphically illustrates the correlation of Infrared peak absorbance at about the 1158 cm$^{-1}$ peak correlating to sulfate species as compared to the ASTM D2896 TBN of various lubricant oils.

## DETAILED DESCRIPTION OF THE INVENTION

The present invention relates to a method for the real time or near real time measurement of a lubricating oil's Total Base Number during engine operation. Specifically, the present invention relates to a method of directly monitoring the TBN of a lubricating oil by measuring one or more pre-selected Infrared ("IR") absorption bands.

Specifically, the present invention has been found to have particular utility in rapidly determining the TBN of a lubricant (if greater than about 20 mg KOH/g) in an operating system comprising:

- Measuring the infrared absorption band of an overbased lubricant from about 840 cm$^{-1}$ to about 910 cm$^{-1}$

- Determining the value of the peak absorbance within that range.

- Calculating the lubricant's TBN from the value of the peak absorbance.

Similarly, the present invention has been found to have particular utility in rapidly determining the TBN depletion of a lubricant provided that TBN depletion from new oil is limited to about 40 mg KOH/g:

- Measuring the infrared band of a lubricant from about 1020 cm$^{-1}$ to about 1285 cm$^{-1}$

- Determining the value of the peak absorbance within that range

- Calculating the lubricant's TBN depletion from the value of peak absorbance.

More specifically, the present method has been found to be particularly effective in measuring the TBN of marine diesel lubricants, where overbase carbonate is commonly present.

There are several indirect methods, i.e. methods where an acid reagent is added, to measure an oil's TBN, none of which can easily be employed in an operating engine. The ASTM methods involve the potentiometric titration of the base in the lubricant using either perchloric or hydrochloric acid. Both acids are extremely strong and the results are sometimes difficult to interpret, making the test method difficult even in the laboratory environment. The other methods also require the addition of acid to the lubricant, whereby reaction by-products are measured The addition of acid to the lube oil sample adds complexity to the measurement of TBN. Further, the handling of acid is undesirable from a safety standpoint, and if spilled for any reason, the acid could cause corrosive damage to the engine if the test unit is in close proximity. Also, the addition of an acid degrades lubricant functionality.

The present invention has discovered that the TBN of a lubricant may be *directly* measured using an IR device, i.e. without the addition of an acid reagent. The present invention noted that overbased lubricant additive are produced in a process known as carbonation which produces alkaline earth metal carbonates in the additive. The present method found that the infrared spectra was particularly sensitive in its ability to highlight carbonate species by measuring its IR absorbance at about 863 cm$^{-1}$.

Although there are several absorbance bands due to carbonate present in the IR spectrum, the present invention found particular utility in the peak at about 863 cm$^{-1}$ as it is strong enough to be measured even at a moderate to low TBN, but not so strong as to lose ability to quantify the peak at high TBN. Measurement of the carbonate peak produces an accurate correlation to TBN levels above about 20 mg KOH/g, but below this level, the peak becomes weak and is less accurately measured.

One advantage of this method is that with most lubricants, TBN can be determined with good accuracy without knowing the TBN of the fresh oil. Knowing the absolute absorbance of the, carbonate band is sufficient to determine TBN. In an overbased lubricant detergent, the components of the total TBN of the overbased detergent are from the metal detergent and from the overbased carbonation. So long as the contribution to TBN from the overbasing is at least two times the contribution of the other components contributing to TBN, this method will determine TBN in the lubricant with good accuracy. Better accuracy is obtained with higher ratios of carbonate TBN to the TBN contributed by other components. Preferably, a ratio of at least three to one is used. More preferably, the precise ratio is known providing for the most accurate measurement. One of ordinary skill in the art would know how to determine the various contributions to TBN.

Below the two to one ratio, the TBN may be accurately determined by employing a difference spectrum to the fresh lubricant with known TBN and measuring the loss of carbonate. Typically, marine diesel engine lubricants, particularly crosshead diesel cylinder oils, are highly overbased and far exceed the two to one ratio, even where products with different formulations are used.

The present invention also discovered that the TBN depletion of a lubricant may be accurately measured by directly measuring the band of the IR spectrum at about 1160 cm-1 which correlates to the sulfate present. Sulfate, usually calcium sulfate, is a by-product of the sulfuric acid neutralization of carbonate overbase, usually calcium carbonate, and the other base containing additives. The present method found that the infrared band for sulfate could be easily quantified by measuring the intensity of the peak at about 1160 $cm^{-1}$.

Although there are several sulfate absorbance bands present in the IR spectrum, the present invention found particular utility in the peak at about 1160 $cm^{-1}$ as it is strong enough to be measured even at low TBN depletion. However, the intensity of this band is such that it can be accurately quantified only if the TBN depletion from new oil is no more than about 40 mg KOH/g using a 0.10 mm path length liquid cell. According to Beer's Law where absorbance is proportional to the pathlength of the cell, this limitation can be avoided by using a liquid cell with a narrower path length, but this is not recommended for dirty oil samples. Alternatively, weak sulfate bands at about 600 and 660 $cm^{-1}$ may also be used when the TBN depletion is very large.

One of ordinary skill in the art would instantly recognize that these two methods may be combined to determine the TBN of any oil. The depletion method allows a practitioner to determine low TBN's below the direct resolution of the carbonate method, provided that the nominal TBN of the fresh oil is known.

Example 1

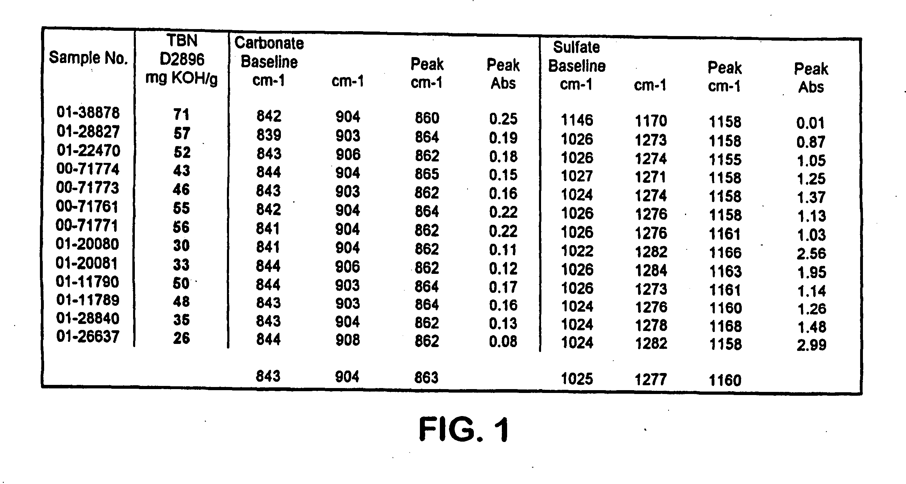

The following non-limiting example demonstrates the current invention. IR spectra of twelve scrapedown (used) cylinder oils from crosshead diesel engines and of a fresh oil sample (70 TBN) were acquired using a Mattson Research Series Fourier Transform Infrared (FTIR) spectrometer. Eight scans were made on each sample at a resolution of 4 $cm^{-1}$. Samples were run in a 0.1 mm path length barium fluoride liquid cell. The results are listed in Figure 1. The samples in this example range in TBN from 26 to 71 mg KOH/g, as measured by D2896. Information concerning the details of FTIR instrumentation and the procedures on how it is used are provided in Coates, J., Analytical Instrumentation Handbook, G. W. Ewing, ed., Marcel Dekker, 1990, Chpt. 7.

Absorbance measurements were made for each spectrum by drawing a straight baseline between the frequencies indicated and measuring the peak intensities relative to the baseline, known herein as the "peak absorbance". The average peak absorbance frequency for carbonate was measured at 863 $cm^{-1}$, and for sulfate was measured at 1160 $cm^{-1}$.

Determining the baseline for the carbonate and sulfate bands is important because they are not parallel to the spectrum X-axis in this region and they typically are above the zero absorbance level. This is largely due to the fact that the salt windows absorb IR radiation, particularly at low frequencies and the base oil, the additive components and contaminants often contribute to a broad absorption band which overlaps with the absorption bands of interest.

Calculating a baseline near the extreme ends of a peak is a convenient way of subtracting out undesirable interferences. A baseline may be constructed by determining a frequency range of interest, and drawing a line between those endpoint frequencies. The "net peak absorbance" is the absolute absorbance at the peak frequency in that range less the calculated "baseline" absorbance at that frequency.

As an example of this method, let the IR spectrum have an absorbance of $\alpha_0$ at frequency $\lambda_0$ at one endpoint of the range, and $\alpha_1$ at $\lambda_1$ at the other endpoint of the range. Let the frequency at which peak absorbance for that range occurs be at $\lambda_p$ which has an absolute absorbance of $\alpha_p$ at the peak and $\alpha_b$ at the calculated baseline. Then the "net peak absorbance", $\alpha_{Peak}$, is determined by the equations:

$$\alpha_b = \alpha_0 + \left[\frac{\alpha_1 - \alpha_0}{\lambda_1 - \lambda_0}\right]\left(\lambda_p - \lambda_0\right)$$

$$\alpha_{Peak} = \alpha_p - \alpha_b$$

For these equations, it is assumed that the frequency scale is linear. However, one of ordinary skill in the art instantly recognizes that these equations may be modified to work on other scaling, such as exponential or logarithmic.

Although the above method is preferred because it does not require a reference sample, another way of minimizing interference in calculating the "net peak absorbance" is to take a difference spectrum and make measurements on it. A difference spectrum in this context is where a spectrum of a fresh oil is subtracted from the spectrum of a used oil. Thus the "net peak absorbance" would be the difference between the absorbance at the peak frequency of the used oil against the absorbance at that same frequency on the fresh oil. Either way, calculated baselines are commonly used to quantify absorption bands of interest.

The results of these measurements are tabularly presented in Figure 1. Line fits through the data were made using the Levenberg-Marquardt algorithm for straight line fits which is described in W. H. Press, et al., Numerical Recipes in C, Cambridge University Press, 1993.

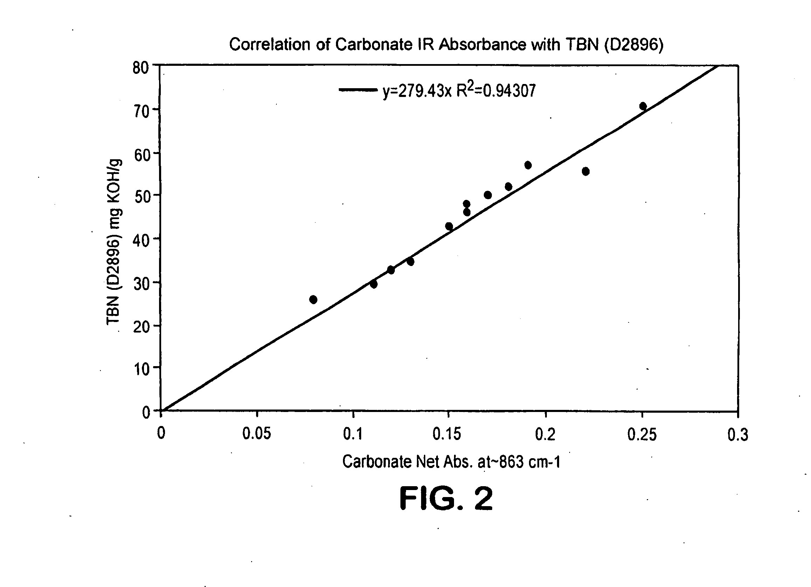

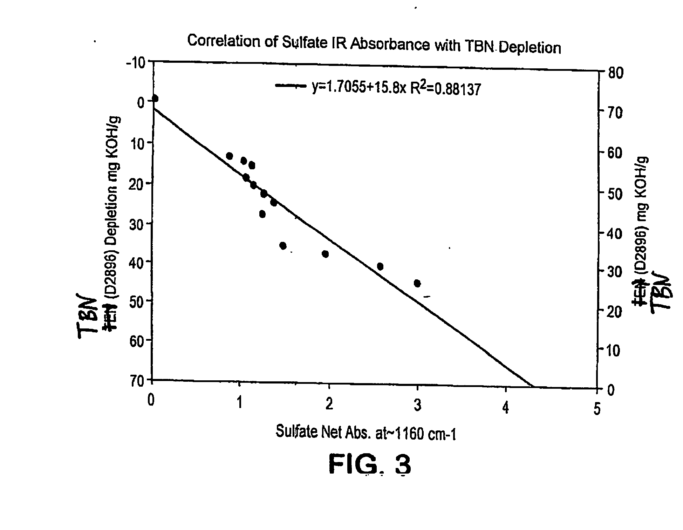

They are graphically presented in Figures 2 and 3.

Figure 2 is a plot of the carbonate net peak absorbance versus TBN (D2896) for each of the thirteen samples analyzed in Example 1. This plot demonstrates that above 20 TBN there is a significant correlation between the value of the carbonate absorbance at about 863 cm$^{-1}$ and the ASTM D2896 TBN of the lubricant. Specifically, a statistical analysis of the data showed a .94 correlation ($R^2$) that the TBN of an oil was linearly related to the peak intensity at about the 863 cm$^{-1}$ frequency. The linear coefficient was equal to approximately 279 mg kOH/g.

Figure 3 is a plot of the sulfate net peak intensities absorbance versus TBN depletion for each of the thirteen samples analyzed in Example 1. The TBN depletion was calculated by subtracting the ASTM D2896 TBN from the known 70 TBN of the fresh samples. This plot demonstrates that there is a significant correlation between the value of the sulfate absorbance at about 1160 cm$^{-1}$ and the TBN of the lubricant over a depletion range of about 40 mg KOH/g. Specifically, a statistical analysis of the data showed a .88 correlation ($R^2$) that the TBN of an oil was linearly related to the absorbance at about the 1160 cm$^{-1}$ frequency. The linear coefficient was equal to approximately -15.8 mg KOH/g.

While this particular experiment was performed on scrape down cylinder oils, the method is applicable to any lubricating oil for which TBN is a useful property. Preferably, this method is particularly useful for measuring TBN in lubricants which are once-through, i.e. the used lubricant goes to waste rather than to a sump after lubricating the cylinder.

**Claims**

1.  A method to determine the Total Base Number (TBN) of a lubricant with a TBN greater than about 20 mg KOH/g **characterised in** comprising:

    a) measuring the infrared absorption band for carbonate species ranging from 840 to 910 cm$^{-1}$,
    b) determining the value of absorbance for at least one peak of interest within said band frequency, and
    c) calculating said lubricant's TBN from the value of the peak absorbance from a calculated baseline.

2.  A method as in claim 1, wherein the frequency of said peak of interest occurs between 860 to 865 cm$^{-1}$.

3.  A method as in claim 2, wherein the frequency of said peak of interest is 863 cm$^{-1}$.

4.  A method as in any of claims 1 to 3, wherein said lubricant's TBN is linearly related to said value of peak absorbance from the calculated baseline.

5.  A method to determine the Total Base Number depletion of a lubricant caracterised in comprising:

    a) measuring the infrared absorption band for sulfate species within a range from 1020 cm$^{-1}$ to 1285 cm$^{-1}$, from 570 to 630 cm$^{-1}$ or from 630 to 690 cm$^{-1}$,
    b) determining the value of absorbance for at least one peak of interest within said range of frequencies,
    c) calculating said lubricant's TBN depletion from said value of the peak absorbance from a calculated baseline.

6.  A method as in claim 5, wherein the frequency of said peak of interest occurs between about 1150 and about 1170 cm$^{-1}$.

7.  A method as in claim 6, wherein the frequency of said peak of interest occurs at about 1160 cm$^{-1}$.

8.  A method as in any one of claims 5 to 7, wherein said lubricant's TBN depletion is linearly related to said value of peak absorbance from the calculated baseline.

9.  A method as in any one of claims 1 to 8, wherein the value of the lubricant's TBN is determined for lubricant in or near an operating engine.

10. A method as in claim 9, wherein said engine is a two-stroke diesel engine.

**Patentansprüche**

1.  Verfahren zur Bestimmung der Gesamtbasenzahl (TBN) eines Schmierstoffs mit einer TBN größer als etwa 20 mg KOH/g,
    **dadurch gekennzeichnet, dass**

    a) die Infrarotabsorptionsbande für Carbonatspezies im Bereich von 840 bis 910 cm$^{-1}$ gemessen wird,

b) der Wert der Extinktion für mindestens ein interessierendes Signal innerhalb der Bandfrequenz bestimmt wird und

c) die TBN des Schmierstoffs aus dem Wert der Signalextinktion gegenüber einer berechneten Basislinie berechnet wird.

2. Verfahren nach Anspruch 1, bei dem die Frequenz des interessierenden Signals zwischen 860 und 865 cm$^{-1}$ liegt.

3. Verfahren nach Anspruch 2, bei dem die Frequenz des interessierenden Signals 863 cm$^{-1}$ beträgt.

4. Verfahren nach einem der Ansprüche 1 bis 3, bei dem die TBN des Schmierstoffs linear mit dem Wert der Signalextinktion gegenüber der berechneten Basislinie verknüpft ist.

5. Verfahren zur Bestimmung der Gesamtbasenzahlverringerung eines Schmierstoffs, **dadurch gekennzeichnet, dass**

a) die Infrarotabsorptionsbande für Sulfatspezies in einem Bereich von 1020 cm$^{-1}$ bis 1285 cm$^{-1}$, von 570 bis 630 cm$^{-1}$ oder von 630 bis 690 cm$^{-1}$ gemessen wird,

b) der Wert der Extinktion für mindestens ein interessierendes Signal innerhalb der Frequenzbereiche bestimmt wird und

c) die TBN-Verringerung des Schmierstoffs aus dem Wert der Signalextinktion gegenüber einer berechneten Basislinie berechnet wird.

6. Verfahren nach Anspruch 5, bei dem die Frequenz des interessierenden Signals zwischen etwa 1150 und etwa 1170 cm$^{-1}$ liegt.

7. Verfahren nach Anspruch 6, bei dem die Frequenz des interessierenden Signals bei etwa 1160 cm$^{-1}$ liegt.

8. Verfahren nach einem der Ansprüche 5 bis 7, bei dem die TBN-Verringerung des Schmierstoffs linear mit dem Wert der Signalextinktion gegenüber der berechneten Basislinie verknüpft ist.

9. Verfahren nach einem der Ansprüche 1 bis 8, bei dem der Wert der TBN des Schmierstoffs für Schmierstoff in oder nahe einem laufenden Motor bestimmt wird.

10. Verfahren nach Anspruch 9, bei dem der Motor ein Zweitakt-Dieselmotor ist

## Revendications

1. Procédé permettant de déterminer l'indice d'alcalinité totale (TBN) d'un lubrifiant ayant un TBN supérieur à environ 20 mg de KOH/g, **caractérisé en ce qu'**il comprend les étapes consistant à :

a) mesurer la bande d'absorption infrarouge pour l'espèce carbonate allant de 840 à 910 cm$^{-1}$,

b) déterminer la valeur de l'absorbance pour au moins un pic présentant un intérêt à l'intérieur de ladite fréquence de bande, et

c) calculer ledit TBN du lubrifiant d'après la valeur de l'absorbance du pic à partir d'une ligne de base calculée.

2. Procédé selon la revendication 1, dans lequel la fréquence dudit pic présentant un intérêt apparaît entre 860 et 865 cm$^{-1}$.

3. Procédé selon la revendication 2, dans lequel la fréquence dudit pic présentant un intérêt est de 863 cm$^{-1}$.

4. Procédé selon l'une quelconque des revendications 1 à 3, dans lequel le TBN dudit lubrifiant est en relation linéaire avec ladite valeur de l'absorbance du pic à partir de la ligne de base calculée.

5. Procédé permettant de déterminer la perte d'indice d'alcalinité totale d'un lubrifiant, **caractérisé en ce qu'**il comprend les étapes consistant à :

a) mesurer la bande d'absorption infrarouge pour l'espèce sulfate sur une gamme de 1 020 cm$^{-1}$ à 1 285 cm$^{-1}$, de 570 à 630 cm$^{-1}$ ou de 630 à 690 cm$^{-1}$,

b) déterminer la valeur de l'absorbance pour au moins un pic présentant un intérêt dans ladite gamme de fréquences,

c) calculer la perte de TBN dudit lubrifiant d'après ladite valeur de l'absorbance du pic à partir d'une ligne de base calculée.

6. Procédé selon la revendication 5, dans lequel la fréquence dudit pic présentant un intérêt apparaît entre environ 1 150 et environ 1 170 cm$^{-1}$.

7. Procédé selon la revendication 6, dans lequel la fréquence dudit pic présentant un intérêt apparaît à environ 1 160 cm$^{-1}$.

8. Procédé selon l'une quelconque des revendications 5 à 7, dans lequel la perte de TBN dudit lubrifiant est en relation linéaire avec ladite valeur de l'absorbance du pic à partir de la ligne de base calculée.

**9.** Procédé selon l'une quelconque des revendications 1 à 8, dans lequel la valeur du TBN du lubrifiant est déterminée pour le lubrifiant à l'intérieur ou au voisinage d'un moteur en fonctionnement.

**10.** Procédé selon la revendication 9, dans lequel ledit moteur est un moteur diesel à deux temps.

| Sample No. | TBN D2896 mg KOH/g | Carbonate Baseline cm-1 | cm-1 | Peak cm-1 | Peak Abs | Sulfate Baseline cm-1 | cm-1 | Peak cm-1 | Peak Abs |
|---|---|---|---|---|---|---|---|---|---|
| 01-38878 | 71 | 842 | 904 | 860 | 0.25 | 1146 | 1170 | 1158 | 0.01 |
| 01-28827 | 57 | 839 | 903 | 864 | 0.19 | 1026 | 1273 | 1158 | 0.87 |
| 01-22470 | 52 | 843 | 906 | 862 | 0.18 | 1026 | 1274 | 1155 | 1.05 |
| 00-71774 | 43 | 844 | 904 | 865 | 0.15 | 1027 | 1271 | 1158 | 1.25 |
| 00-71773 | 46 | 843 | 903 | 862 | 0.16 | 1024 | 1274 | 1158 | 1.37 |
| 00-71761 | 55 | 842 | 904 | 864 | 0.22 | 1026 | 1276 | 1158 | 1.13 |
| 00-71771 | 56 | 841 | 904 | 862 | 0.22 | 1026 | 1276 | 1161 | 1.03 |
| 01-20080 | 30 | 841 | 904 | 862 | 0.11 | 1022 | 1282 | 1166 | 2.56 |
| 01-20081 | 33 | 844 | 906 | 862 | 0.12 | 1026 | 1284 | 1163 | 1.95 |
| 01-11790 | 50 | 844 | 903 | 864 | 0.17 | 1026 | 1273 | 1161 | 1.14 |
| 01-11789 | 48 | 843 | 903 | 864 | 0.16 | 1024 | 1276 | 1160 | 1.26 |
| 01-28840 | 35 | 843 | 904 | 862 | 0.13 | 1024 | 1278 | 1168 | 1.48 |
| 01-26637 | 26 | 844 | 908 | 862 | 0.08 | 1024 | 1282 | 1158 | 2.99 |
| | | 843 | 904 | 863 | | 1025 | 1277 | 1160 | |

## FIG. 1

EP 1 485 696 B1

**Correlation of Carbonate IR Absorbance with TBN (D2896)**

y=279.43x R$^2$=0.94307

TBN (D2896) mg KOH/g

Carbonate Net Abs. at~863 cm-1

## FIG. 2

Correlation of Sulfate IR Absorbance with TBN Depletion

$y=1.7055+15.8x \quad R^2=0.88137$

Sulfate Net Abs. at~1160 cm-1

**FIG. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5250204 A
- EP 695798 A
- GB 2180338 A
- US 5470495 A
- US 478224 A
- US 5569842 A

### Non-patent literature cited in the description

- Kirk-Othmer Encyclopedia of Chemical Technology. vol. 8, 39
- **COATES, J.** Analytical Instrumentation Handbook. Marcel Dekker, 1990
- **W. H. PRESS et al.** Numerical Recipes in C. Cambridge University Press, 1993